# EUROPEAN PATENT SPECIFICATION

(11) **EP 1 350 493 B1**
(45) Date of publication and mention of the grant of the patent: **26.03.2008**
(21) Application number: 02007498.5
(22) Date of filing: 02.04.2002
(51) Int. Cl.: A61F 13/15

(54) **Absorbent product side flap arrangement**
Seitenklappeneinrichtung für absorbierenden Artikel
Système de rabats latéraux pour produit absorbant

(43) Date of publication of application: 08.10.2003
(73) Proprietor: SCA Hygiene Products AB, 405 03 Göteborg (SE)
(72) Inventor: Johansson, Mikael, 432 42 Varberg (SE)
(74) Representative: Bergstrand, Mikael Gudmundsson

(56) References cited:
- WO-A-96/21412
- WO-A-98/29080
- FR-A- 2 734 712
- GB-A- 2 292 067
- US-A- 5 531 729
- US-A- 5 624 420

## Description

### Field of the invention:

The present invention relates to an absorbent product for absorbing human exudate, such as a diaper, having side flaps at one end thereof to allow for releasable closure of said product around a user's waist. In particular, the invention relates to absorbent products having elastic side flaps, and to absorbent products of the disposable type, i.e. of the type which are to be discarded when soiled.

The invention may however also be used with other absorbent products such as training pants which comprise sides which can be opened and then re-closed, or with products comprising re-usable absorbent core elements for example.

### Background to the invention:

Disposable absorbent products having elastic side flaps are well known. One example of such an absorbent product having the features defined in the preamble of claim 1 is a diaper as disclosed in the document WO-A-99/60967. This document discloses an elastically extensible waist region comprising a continuous belt. Additionally disclosed, distinct from the elastic in the waist section, are side flaps which may be elastic. The elasticity is provided by means of a layered structure consisting of a further elastic member, preferably laminated between layers of the absorbent product chassis. In the described product, the elastic in the waist portion also extends continuously into the upper portion of the side flaps.

When the side flaps are made elastic in WO-A-99/60967, the elastic material extends over the whole area of the side flaps from its lower edge upwards, although it is kept separate from the elastic material which is present in the waist region.

Even though the product known from WO-A-99/60967 has the advantage of being able to provide elasticated side flaps, such a construction is expensive due to the amount of elastic material used, elastic material being considerably more expensive than other sheet layer material (e.g. nonwoven material or other polymeric materials) used in non-elasticated side flaps and other non-elastic parts of absorbent products. Moreover, the structure of the side flaps is complicated when the side flaps should be formed to be elastic, since both waist elastic and separate side elastic are used.

US 5,531,729 discloses an absorbent article, such as a diaper or is continence briefs, having side-flaps which are integral with the back sheet and top sheet forming the body of the product. The article has elastic sections in the upper past (waist) of the product. Separate side flaps are not disclosed.

FR 2 734 712 discloses an absorbant product having separate side flaps, said side flaps being elasticated over the whole area of the same. The elasticated area is not limited to only past of the longitudinal extent of the side flap.

In WO 96/21412, an absorbent article is disclosed having partially elasticated side-panels, wherein the elastic portions are provided at various locations in both upper and lower portions of the longitudinal extent of the side-panels.

Additionally, elastic side flaps present a further disadvantage in that the portion of the absorbent product which is located laterally in line with the longitudinal extent of the elastic side flaps, tends to be bunched together as a result of the elastic forces from the elastic side flaps. This bunching together results in the back of the wearer being less well covered (i.e. the absorbent product is less well fitted/adapted to the back of the wearer) as compared to when non-elastic side flaps are used.

Since material cost is a primary concern in absorbent products, it is an object of the invention to provide an absorbent product having elasticated side flaps whereby a simple structure is obtained and whereby material costs are minimized without unduly affecting the functionality of the product as regard comfort and leakage etc.

It is an additional object of the invention to provide an elasticated side flap structure which improves the fit of the diaper to the back of the wearer.

### Summary of the invention

The object of the invention is achieved by an absorbent product having the features defined in claim 1. Preferred features of the invention are defined in the dependent claims.

Further features of the invention will be apparent to the reader of this specification.

In accordance with the invention, the absorbent product is defined with respect to a longitudinal axis and a lateral axis. In this regard a longitudinal axis is intended to be that axis which extends between first and second opposed end portions which are joined by a middle or crotch portion. The mid points of the first, second and crotch portions are thus generally aligned with the longitudinal axis in most cases, unless non-uniformly arranged products are selected. The lateral axis is an axis intended to define the lateral or transverse direction and is thus generally perpendicular to, and normally substantially perpendicular to, the longitudinal axis.

While the invention is defined in relation to a first end portion, it will be understood that the first end portion may be chosen to lie at either end of the product.

The first end portion, which may be the rear portion intended to be worn at the wearer's back during use, comprises two side flaps on either side of a waist section. The waist section itself may be provided with waist elastic to provide additional comfort, whereby any elastic provided in the waist section should allow the waist section to be able to be extended elastically by up to 50% and preferably up to about 100%. However, the invention is primarily concerned with the elastic section of the side flaps which lies outside any elastic present in the waist region.

In the side flaps however, the elasticity should be greater than that in the waist section lying between the side flaps. The side flaps should thus be elastically extensible by an amount of more than 100% and preferably more than 150%, more preferably by more than 250% and even more preferably by more than 300%.

An elastic section is considered to be elastic in accordance with the invention if it can be elastically extended by more than 50% without substantial permanent strain, whereby substantial permanent strain is understood as being of the order of more than about 10 to 30% of the initial length at the first stretching.

In the invention, the elastic section of the side flap is to be understood as that part of the side flap, distinct from any elastic in the waist section, which is provided with elastic material allowing the section to be stretched elastically, at least in the lateral direction. Thus, the lower border of the elastic section should be understood as the lower edge of where the elastic material (e.g. rubber sheet or elastic film material or the like) is located in the side flap.

Non-elastic sections may comprise a continuous or discontinuous sheet, web, layer or fabric for example. A non-elastic section can be stretched elastically by only small amounts, normally of the order of between 1 to 15%, but typically less than 10%.

Side flaps in accordance with the invention are used in order to attach the first end portion of the absorbent product to the second end portion in order to provide a closed absorbent product. For this purpose, the side flaps are provided with releasable attachment means, preferably in the form of a releasable mechanical fastener of the hook and loop type (e.g. Velcro®). Releasable mechanical fasteners which may be used with the absorbent product of the invention refer to the type of fasteners which can be attached, removed and reattached by simple and slight pressure, but are not intended to include press-studs and buttons.

The side flaps are discrete portions which are fixedly attached, e.g. by welding or adhesive, to the chassis of the absorbent product, whereby the chassis should be understood as being that part of the product which will contain the absorbent core. Typically the chassis will be formed by the liquid permeable top sheet and the liquid impermeable back sheet and any further sheets therebetween, together with an absorbent core sandwiched between the top sheet and back sheet.

Furthermore the side flaps are preferably used to form the outermost parts of the product which are to conform to the leg opening portions of the product which are formed when the first and second end portions are releasably attached. Thus, in order to provide close conformability to the legs, the lower edge of the side flaps will preferably be curved, substantially starting from a point where the side flaps intersect the longitudinal side margins of the chassis and up to the lowermost point on the side flap outer edge.

### Brief description of the drawings:

The invention will now be explained in more detail with reference to certain non-limiting embodiments thereof and with the aid of the accompanying drawings, in which:
- Fig. 1: shows an absorbent product in accordance with the invention in a laid flat condition,
- Fig. 2: shows a cross-sectional view along line II-II in Fig. 1,
- Fig. 3: shows a partial view depicting a corner of an absorbent product including an alternative embodiment of the invention,

- Fig. 4: shows an exemplary plan view illustrating a method of applying a pair of side flaps on one side of an absorbent product.

### Detailed description of preferred embodiments:

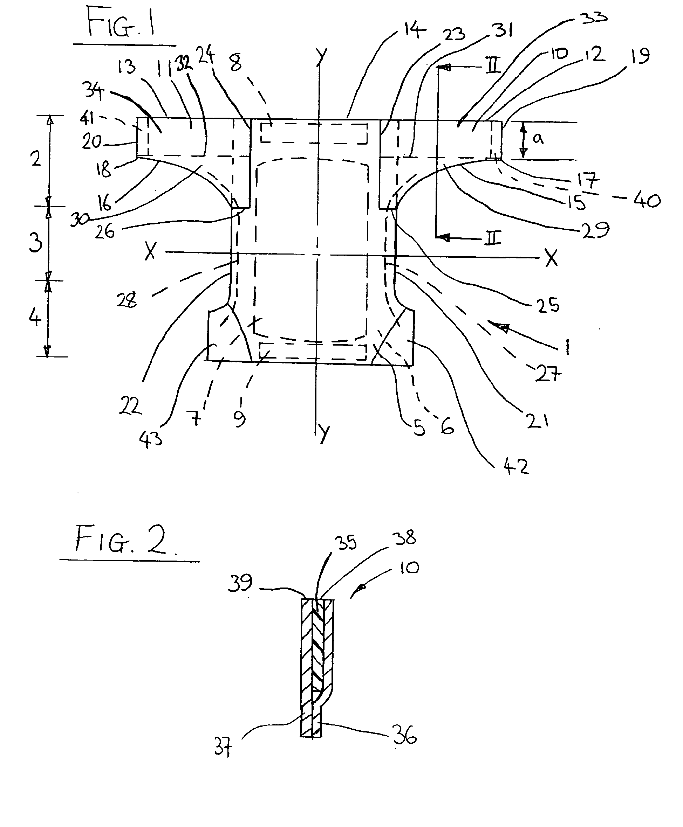

In Fig. 1, the absorbent product 1 comprises a longitudinal axis Y-Y and a lateral axis X-X extending generally perpendicular thereto. In the longitudinal direction, the absorbent product 1 is divided into three portions, a first end portion 2 a middle or crotch portion 3, and a second end portion 4. The first end portion 2 is, in the shown embodiment, intended to be the rear portion of the diaper, i.e. that portion of the absorbent product intended to be located against the wearer's back during use.

The longitudinal axis Y-Y thus passes generally through the mid points of each of these three portions 2, 3, 4.

The absorbent product is shown in plan view with a liquid impermeable back sheet 5 shown uppermost. A liquid permeable top sheet 6 is placed on the opposite side of the back sheet 5 and is preferably co-extensive therewith and sealed to the back sheet 5 at the peripheries of said sheets, e.g. by welding or adhesive (not shown). In between the back sheet 5 and the top sheet 6 is placed an absorbent core 7. The possible structures and materials of the top sheet 6, back sheet 5 and absorbent core 7 can be widely varied, and many possibilities are known in the art. No further description of same is given in this specification, since appropriate structures and materials will be apparent to a skilled person.

In the first end portion 2 and the second end portion 4, there is preferably a first elasticated waist section 8 and a second elasticated waist section 9 respectively. The waist elastic, where present, preferably allows an extension of up to 50% to allow the user's movements, when standing and sitting for example, to be taken into account whilst still providing a comfortable and well-fitting waist portion. The waist elastic, when present, is preferably located outside the longitudinal limits of the absorbent core 7 so that any bunching together of the absorbent core 7 by the elastic forces in the waist elastic is minimized.

On each side of the first waist section 8 are located a first side flap 10 and a second side flap 11. The side flaps 10, 11 are preferably substantially identical. Most preferred is the arrangement depicted, whereby the first and second side flaps 10, 11 are identical mirror images of one another.

Each side flap 10, 11 has an upper edge 12, 13 located in the shown embodiment substantially in line with the upper edge of the chassis member (i.e. the top edge 14 of the combined top sheet 5 and back sheet 6 in this case). The lower edge 15, 16 of each flap has a curved portion extending from the lowest point 17, 18 of the outer edge 19, 20. The curved portion intersects the chassis along the longitudinal side edges 21, 22 and has a small portion 25, 26 extending laterally up to the inner edge 23, 24.

Longitudinal side edges 21, 22 extend longitudinally from the upper edge 14 of the product and merge into ears or side flaps in the second end portion 4. Leg elastics 27, 28 are positioned proximate the longitudinal side edges 21, 22 of the product and serve to elasticate the leg portions, especially in the crotch region where leakage is most likely to occur. Additionally, the leg elastics preferably extend into a lower non-elastic portion 29, 30 of the side flaps 10, 11.

The lower non-elastic portions 29, 30 respectively extend all the way from the inner edge 23, 24 to the outer edge 19, 20 between the flap lower edge formed by portions 15, 25 and 16, 26 respectively to the lower border 31, 32 of elastic sections 33, 34.

The elastic sections 33, 34 of the side flaps 10, 11 extend, in this embodiment, from the upper edge of the flaps 10, 11 all the way between the inner edge 23, 24 and the outer edge 19, 20 down to the lower border 31, 32 which is aligned with the lower end of the side flap outer edge 17, 18. Thus, the elastic sections in this embodiment are substantially rectangular.

As can be seen, the elastic sections 33, 34 each extend, in a longitudinal direction, only part of the distance between the upper edges 12, 13 and lower edges 15, 25, 16, 26 of the side flaps. The extension of the elastic sections being over only part of the distance is maintained over almost the entire lateral extent (the lateral extent being the lateral distance between the inner edges 23, 24 and outer edges 19, 20) of the side flap. Thus, it can be stated that at least over a portion of the lateral extent of the side flap (preferably over the majority of the lateral extent and more preferably over the entire extent of the flap) the elastic section 33, 34 will extend (in a longitudinal direction) over only part of the distance between said upper edge and said lower edge, and in that a non-elastic section (29, 30) extends between a lower border (31, 32) of said elastic section (33, 34) and said lower edge (15, 25, 16, 26).

The elastic sections 33, 34 each comprises an elastic material 35 such as an elastic lamina (see Fig. 2). For reasons of comfort and use, the elastic material 35 is located adjacent and fixedly attached to at least one layer (e.g. layer 36), or more preferably between two laminae or layers 36, 37 of a non-elastic material. The fixed attachment of the elastic material 35 to the layer(s) can be performed for example by means of adhesive and/or welding.

A preferred material for layers 36 and/or 37 is a non-elastic fibrous material, preferably a nonwoven material made of either continuous filaments (spunbonded) or carded staple fibres (thermobonded, through-air bonded or hydroentangled). Also, nonwoven made of meltblown short fibres may be used. Additionally, nonwovens made by a combination of these technologies may be used. A nonwoven material such as S-M-S material (spunbond-meltblown-spubond material) may be used for example.

In order that the flap is elasticated by use of the elastic material, the elastic material 35 may for example first be stretched to its required lateral extent in use (e.g. 200%) and then attached in its stretched state to one or more non-elastic material layers, such as layers 36, 37. Thus each of the non-elastic layers 36, 37 may extend over the whole area of the side flap 10, 11, while the elastic material 35 only extends in the area which is to form the elastic section 33, 34. Since significant areas of the side flaps now lack elastic material, which is expensive, large cost savings can be made.

Since however non-elastic nonwoven material may elongate when a pulling force is applied (even though the elastic recovery is very low), the elastic material does not have to be fixed under its maximum elongation in order to achieve sufficient elastic properties in the side flap. Certain non-elastic nonwoven materials can for example have a very high rate of extensibility in the lateral direction, such that if these highly extensible yet non-elastic nonwoven materials are used as layer 36 and/or layer 37, the elastic material can even be fixed at zero stretch to the non-elastic material.

Other ways of using elastic material to elasticate the side flaps are also known in the art, such as the use of an activatable elastic material which is non-elastic until heated. Upon heating, the material becomes elastic. Such materials are known in the art and thus not described further here. Such materials may also be used in this invention.

As will be understood from the aforegoing, the lower edges 15, 16, which form at least part of the leg openings, will thus be non-elastic but will still be gathered when the elastic zones 33, 34 are in a relaxed condition. However, it has been found that the use of elastic in this area is not generally required to give good comfort and fit, and the general extension of the side flaps 10, 11 in order to close the waist area of the product is generally sufficient to accomplish this purpose. However, at the area proximate where the lower edges 15, 16 of the side flaps 10, 11 intersect the longitudinal edges 21, 22, the leg elastics 27, 28 may be extended into the side flaps and fixedly attached to the non-elastic sections 29, 30 of the side flaps 10, 11. Even though some elastic material is then present in the non-elastic sections of the side flaps, the leg elastics are however generally strands of moulded rubber (e.g. of the type known as Fullflex®), or elastic film which have a width of some 2 to 6 mm, thus making these considerably cheaper when compared to making the entire side flaps elastic as in prior art solutions.

The leg elastics 27, 28, when present, may extend several cm, e.g. between one and eight cm, into the side flaps. These leg elastics 27, 28 should preferably follow the curvature of the lower edges 15, 25, 16, 26 over at least part of their length and at least over part of the curved portion.

The inner edges 23, 24 of the side flaps 10, 11 preferably lie laterally inwardly of the longitudinal edges 21, 22 to provide an overlap, typically by an amount of between 0.5 and 6 cm. The length of inner edges 23, 24 in the longitudinal direction may lie for example between 4 cm up to 25 cm. The overlap is used to provide a joining area of the chassis (i.e. the joined top 6 and back sheet 5 in this case) to the side flaps 10, 11. In the embodiment shown, the side flaps 10, 11 are each affixed to the outwardly facing side of the back sheet 5. However, it will be apparent that the side flaps 10, 11 may be positioned such that they are affixed to the top sheet 6, or to both the top sheet 6 and the back sheet 5. The side flaps 10, 11 may be joined for example such that each side flap 10, 11 is held between the top sheet 6 and back sheet 5, or such that the top sheet 6 and back sheet 5 are themselves sandwiched between, and fixedly attached to, inner edge portions (i.e. overlapped portions) of separate layers of material (e.g. layers 36, 37) making up the side flaps. Similarly, embodiments are possible where only one of the layers of the side flaps 10, 11 is fixedly attached between the top sheet 6 and back sheet 5, while another layer of the side flap 10, 11 could be attached to an outwardly facing surface of the top sheet 6 or back sheet 5.

On the outer edge portion of side flaps 10, 11 are positioned a first part 40, 41 of a releasable fastening means, in particular a mechanical releasable fastening means (e.g. a hook or loop tab member) . The means is on the lower (inner) side of the side flap 10, 11 in the view shown in Fig. 1. At the second end portion 4 of the absorbent product 1, there are two portions 42, 43 (e.g. of loop material, nonwoven material or hook member material) which co-operate with a respective one of said fastening means 40, 41. Such releasable attachments are well known to the skilled person and thus no further detailed description is provided here.

The two portions 42, 43 could however be replaced by a single attachment portion extending over the central part of the second end portion 4.

Fig. 2 shows the side flap 10 in cross-section, whereby a laminate structure is present. The central material, or central lamina, is an elastic material 35 having an upper edge or border 38. In the embodiment shown, the longitudinal distance "a" (cf. Fig. 1) is equal to the longitudinal length of the elastic material, which is constant throughout the side flap of this embodiment and thus corresponds substantially to the length of outer edge 19, 20. The distance "a" will typically lie between 4cm and 12 cm. The elastic material 35 is sandwiched between and fixed to layers 36, 37 of non-elastic material, such as nonwoven material. The non-elastic material layer(s) preferably extend over the whole surface area of the side flaps 10, 11.

Material layers 36, 37 are shown as single laminae or plies, although it should be understood that each of these layers may comprise more than one lamina or ply (such as in the case of S-M-S materials mentioned above). The various layers may be held together by any suitable means such as welding, in particular ultrasonic welding, or adhesive, or by mechanical methods such as needling, or other methods.

The upper border 38 of the elastic material 35 lies at substantially the same level as the upper edge 12, 13 of the side flap and is co-extensive therewith. In this way the entire area from the upper edge 12, 13 to the lower end points 17, 18 of the outer edges is an elastic section and can be extended elastically.

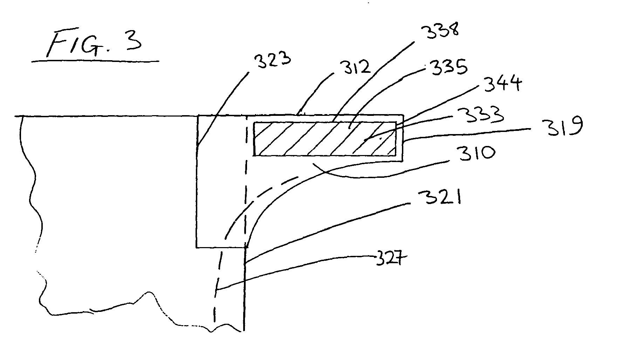

In Fig. 3, a further embodiment is disclosed, in which the elastic material 335 is highlighted. The elastic material 335 is thus used to elasticate the side flap over an area 333. This area 333 extends from a location laterally outwardly of inner edge 323 and also preferably laterally outwardly of longitudinal side edge 321. In this way, use of elastic material is further optimised, since the area of joining of the side flap 310 to the chassis (i.e. the overlap area between edge 323 and edge 321) requires no elastication. In this embodiment, the elastic section 333 does not extend to the upper edge 312 but has its upper border 338 located below same (e.g. 0.3 to 1 cm below edge 312). The side flap 310 however still remains elastic over the majority of its upper area between outer edge 319 and longitudinal side edge 321. The distance between the upper border 338 and the upper edge 312 should however not be too large since otherwise rollover effects at the top edge 312 may occur with resulting lack of comfort. Leg elastic 327 is also shown.

A small border area may also be left between outer edge 344 of the elastic section 333 and the outer edge 319 of the side flap. This border area may be between 0.3 cm and 3 cm for example. The use of a border area means that the outer edge portion of the side flap is not elastically extensible. However, since (as explained in regard to Fig. 1) releasable fastening means (e.g. means 40) are attached at this location, elasticity at this location is not required. Thus further elastic material can be saved.

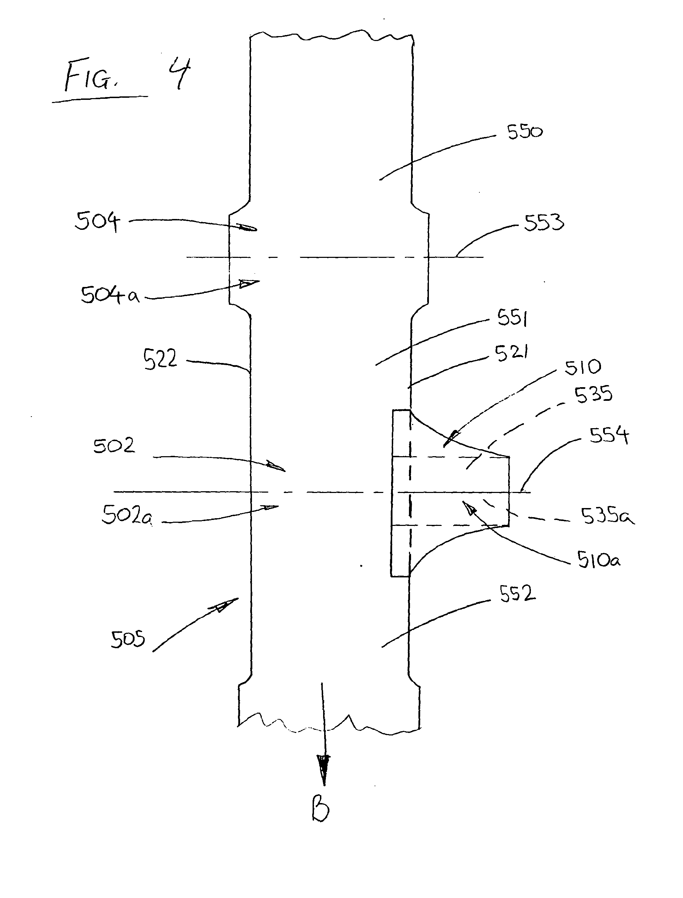

Fig. 4 shows a plan view of a stage of production of the absorbent articles of the present invention. A web of e.g. back sheet 505 material is fed in the direction of arrow B. The part of the web shown depicts absorbent products being made in longitudinal production back-to-back and front-to-front. Thus, line 553 depicts an intended cut line defining the location of two second end portions 504, 504a on either side of the intended cut line 553. Similarly, two first end portions 502, 502a are depicted on either side of an imaginary cut line 554.

A machine (not shown) applies a single piece of fabric comprising two combined side flaps 510, 510a containing a single piece of stretched elastic material here denoted as 535, 535a. The single piece of fabric is laid substantially symmetrically across intended cut line 554, on either side of which cut line 554 two first end portions 502, 502a will be created.

A single piece of fabric is applied to only one side edge 521 in the embodiment shown, although it will be understood that a similar piece of fabric must be applied to the opposite side, at the same location substantially symmetrically about the intended cut line 554 so as to form the side flaps at the opposite side edge 522. Such can occur later, earlier, or at the same time as single fabric piece (comprising 510, 510a) is applied at edge 521.

At a later station (not shown) the web is cut successively at lines 554 and then 553, such that individual products result. The described embodiment is a preferred embodiment of a method for carrying out the invention, which results in time savings due to only single pieces of fabric being required from which two side flaps are made. This also results in a product where the end edges (compare to end edge 14 in Fig. 1) are aligned exactly with the upper edges (compare to edges 12, 13 in Fig. 1) of the side flaps, thus providing a neat and attractive product with no trimming required. However it will be understood that each of the side flaps 510, 510a could be pre-formed as separate pieces and applied and attached separately to the back sheet and/or top sheet of such products.

## Claims

1. Absorbent product (1) for absorbing human exudate, comprising a longitudinal axis (Y-Y) and a lateral axis (X-X) extending generally perpendicular thereto, further comprising a first end portion (2), wherein said first end portion (2) comprises two side flaps (10, 11) each extending laterally outwardly with respect to said longitudinal axis (Y-Y) on either side of a waist section (8), wherein each of said side flaps (10, 11) includes an upper edge (12, 13) and a lower edge (15, 25, 16, 26), said upper and lower edges being separated, and wherein each of said side flaps (10, 11) further comprises an elastic section (33, 34) distinct from any elastic in said waist section (8), wherein at least over a portion of the lateral extent of the side flap (10, 11), said elastic section (33, 34) extends, in a longitudinal direction (Y-Y), over only part of the distance between said upper edge (12, 13) and said lower edge (15, 25, 16, 26), and in that a non-elastic section (29, 30) extends between a lower border (31, 32) of said elastic section (33, 34) and said lower edge (15, 25, 16, 26) **characterized in that** said side flap is a discrete member fixedly attached to the chassis of the absorbent product, said chassis being formed by a liquid permeable top sheet and a liquid impermeable back sheet and any further sheets there between, together with an absorbent core sandwiched between the top sheet and back sheet, said side flap comprising an inner edge (23, 24) and an outer edge (19, 20), said inner edge being longer than said outer edge, and **in that** said non-elastic section (29, 30) extends, longitudinally, from said lower edge (15, 25, 16, 26) up to a level aligned with a lower end point (17, 18) of said outer edge (19, 20) and **in that** said lower edge (15, 25, 16, 25) of said side flap has at least one curved portion (15, 16) extending between said inner edge (23, 24) and said outer edge (19, 20), wherein
each of said elastic sections (33, 34) comprises an elastic laminate including an elastic material (35) located adjacent and fixedly attached to two layers (36, 37) of a non-elastic fibrous material, and wherein
said two layers (36, 37) of a non-elastic fibrous material extend over the whole area of said side flap (10, 11), while the elastic material (35) only extends in the area which is to form the elastic section (33, 34).

2. An absorbent product according to claim 1, **characterized in that** said elastic section (33, 34) extends, longitudinally, substantially the entire distance between the upper edge (12, 13) of said side flap (10, 11) and the lower end point (17, 18) of said outer edge (19, 20).

3. An absorbent product according to claim 1, **characterized in that** said curved portion (15, 16) extends part of the way between said inner edge (23, 24) and the lower end point (17, 18) of said outer edge (19, 20).

4. An absorbent product according to any one of the preceding claims, **characterized in that** each of said side flaps (10, 11) is fixedly attached at a longitudinal side edge (21, 22) portion of said top sheet (6) and/or said back sheet (5) .

5. An absorbent product according to claim 4, **characterized in that** said upper edge (12, 13) is substantially aligned with an upper edge (14) of said top sheet (6) and/or said back sheet (5).

6. An absorbent product according to any one of the preceding claims, **characterized in that** said elastic laminate including an elastic material lamina (35) is fixedly attached in an at least laterally elongated condition between two laminae (36, 37) of a non-elastic fibrous material.

7. An absorbent product according to any one of the preceding claims, **characterized in that** at least one elastic member (27, 28), forming part of an elasticated leg portion on one respective side of said absorbent product, extends into the non-elastic section (29, 30) of said side flap on said respective side.

8. An absorbent product according to any one of the preceding claims, **characterized in that** said elastic section (10, 11) is substantially rectangular, and **in that** said elastic section (10, 11) includes an upper border (38), and **in that** said upper border (38) is aligned with an upper edge (39) of said side flap (10, 11).

## Patentansprüche

1. Absorbierendes Produkt (1) zum Absorbieren menschlicher Exudate, umfassend eine Längsachse (Y-Y) und eine Querachse (X-X), die sich im Wesentlichen senkrecht dazu erstreckt, ferner umfassend einen ersten Endabschnitt (2), wobei der erste Endabschnitt (2) zwei Seitenlaschen (10, 11), die sich in Bezug auf die besagte Längsachse (Y-Y) auf jeder Seite eines Taillenabschnitts (8) seitlich nach außen erstrecken, umfasst, wobei die Seitenlaschen (10, 11) jeweils eine Oberkante (12, 13) und eine Unterkante (15, 25, 16, 26) umfassen, wobei die Ober- und Unterkante getrennt sind und wobei die Seitenlaschen (10, 11) ferner jeweils einen elastischen Abschnitt (33, 34) umfassen, der sich von jeglicher Elastik in dem besagten Taillenabschnitt (8) unterscheidet, wobei sich der elastische Abschnitt (33, 34) wenigstens über einen Abschnitt der seitlichen Erstreckung der Seitenlasche (10, 11) in einer Längsrichtung (Y-Y) über nur einen Teil der Strecke zwischen der Oberkante (12, 13) und der Unterkante (15, 25, 16, 26) erstreckt und sich ein nicht elastischer Abschnitt (29, 30) zwischen einer unteren Grenze (31, 32) des elastischen Abschnitts (33, 34) und der Oberkante (15, 25, 16, 26) erstreckt, **dadurch gekennzeichnet, dass** die Seitenlasche ein separates Element ist, das an der Hülle des absorbierenden Produkts fest angebracht ist, wobei die Hülle durch eine flüssigkeitsdurchlässige Oberlage und eine flüssigkeitsundurchlässige Rücklage und beliebige weitere Lagen dazwischen zusammen mit einem Absorptionskern, der zwischen der Oberlage und der Rücklage angeordnet ist, gebildet ist, wobei die Seitenlasche eine Innenkante (23, 24) und eine Außenkante (19, 20) umfasst, wobei die Innenkante länger ist als die Außenkante und **dadurch, dass** sich der nicht elastische Abschnitt (29, 30) längs von der Unterkante (15, 25, 16, 26) nach oben bis zu einem Niveau in der Flucht mit einem unteren Endpunkt (17, 18) der Außenkante (19, 20) erstreckt und **dadurch, dass** die Unterkante (15, 25, 16, 26) der Seitenlasche wenigstens einen gekrümmten Abschnitt (15, 16) aufweist, der sich zwischen der Innenkante (23, 24) und der Außenkante (19, 20) erstreckt, wobei
die elastischen Abschnitte (33, 34) jeweils ein elastischen Verbund umfassen mit einem elastischen Material (35), das benachbart und an zwei Lagen (36, 37) eines nicht elastischen faserförmigen Materials fest angebracht ist, und wobei
sich die zwei Lagen (36, 37) eines nicht elastischen faserförmigen Materials über die gesamte Fläche der Seitenlasche (10, 11) erstrecken, während sich das elastische Material (35) nur in dem Bereich erstreckt, der den elastischen Abschnitt (33, 34) bilden soll.

2. Absorbierendes Produkt nach Anspruch 1, **dadurch gekennzeichnet, dass** sich der elastische Abschnitt (33, 34) längs im Wesentlichen über die gesamte Strecke zwischen der Oberkante (12, 13) der Seitenlasche (10, 11) und dem unteren Endpunkt (17, 18) der Außenkante (19, 20) erstreckt.

3. Absorbierendes Produkt nach Anspruch 1, **dadurch gekennzeichnet, dass** sich der gekrümmte Abschnitt (15, 16) über einen Teil der Strecke zwischen der Innenkante (23, 24) und dem unteren Endpunkt (17, 18) der Außenkante (19, 20) erstreckt.

4. Absorbierendes Produkt nach einem der vorstehenden Ansprüche, **dadurch gekennzeichnet, dass** die Seitenlaschen (10, 11) jeweils an einem Abschnitt an einer längslaufenden Seitenkante (21, 22) der Oberlage (6) und/oder der Rücklage (5) fest angebracht sind.

5. Absorbierendes Produkt nach Anspruch 4, **dadurch gekennzeichnet, dass** die Oberkante (12, 13) im Wesentlichen mit einer Oberkante (14) der Oberlage (6) und/oder Rücklage (5) fluchtet.

6. Absorbierendes Produkt nach einem der vorstehenden Ansprüche, **dadurch gekennzeichnet, dass** der elastische Verbund umfassend einen elastischen Materialverbund (35) in einem wenigstens seitlich länglichen Zustand zwischen zwei Verbunden (36, 37) aus einem nicht elastischen faserförmigen Material fest angebracht ist.

7. Absorbierendes Produkt nach einem der vorstehenden Ansprüche, **dadurch gekennzeichnet, dass** sich wenigstens ein elastisches Element (27, 28), das einen Teil eines elastischen Beinabschnitts auf einer entsprechenden Seite des absorbierenden Produkts bildet, in den nicht elastischen Abschnitt (29, 30) der Seitenlasche auf der entsprechenden Seite erstreckt.

8. Absorbierendes Produkt nach einem der vorstehenden Ansprüche, **dadurch gekennzeichnet, dass** der elastische Abschnitt (10, 11) im Wesentlichen rechteckig ist und **dadurch, dass** der elastische Abschnitt (10, 11) eine obere Grenze (38) umfasst und **dadurch, dass** die obere Grenze (38) mit einer Oberkante (39) der Seitenlasche (10, 11) fluchtet.

## Revendications

1. Article absorbant (1) pour absorber un exsudat humain, comprenant un axe longitudinal (Y-Y) et un axe latéral (X-X) s'étendant de manière générale perpendiculairement à celui-ci, comprenant en outre une première partie d'extrémité (2), ladite première partie d'extrémité (2) comprenant deux rabats latéraux (10, 11) s'étendant chacun latéralement vers l'extérieur par rapport audit axe longitudinal (Y-Y) de chaque côté d'une section de taille (8), dans lequel chacun desdits rabats latéraux (10, 11) comprend un bord supérieur (12, 13) et un bord inférieur (15, 25, 16, 26), lesdits bords supérieur et inférieur étant séparés, et dans lequel chacun desdits rabats latéraux (10, 11) comprend en outre une section élastique (33, 34) distincte de tout élastique présent dans ladite section de taille (8), dans lequel sur au moins une partie de l'étendue latérale du rabat latéral (10, 11), ladite section élastique (33, 34) s'étend, dans une direction longitudinale (Y-Y), seulement sur une partie de la distance séparant ledit bord supérieur (12, 13) dudit bord inférieur (15, 25, 16, 26), et en ce qu'une section non élastique (29, 30) s'étend entre une limite inférieure (31, 32) de ladite section élastique (33, 34) et ledit bord inférieur (15, 25, 16, 26), **caractérisé en ce que** ledit rabat latéral est un élément discret attaché de manière fixe au châssis de l'article absorbant, ledit châssis étant formé par une feuille supérieure perméable aux liquides et une feuille arrière imperméable aux liquides et n'importe quel nombre de feuilles supplémentaires entre elles, avec un noyau absorbant pris en sandwich entre la feuille supérieure et la feuille arrière, ledit rabat latéral comprenant un bord intérieur (23, 24) et un bord extérieur (19, 20), ledit bord intérieur étant plus long que ledit bord extérieur, et **en ce que** ladite section non élastique (29, 30) s'étend, dans le sens longitudinal, dudit bord inférieur (15, 25, 16, 26) à un niveau aligné avec un point d'extrémité inférieure (17, 18) dudit bord extérieur (19, 20) et **en ce que** ledit bord inférieur (15, 25, 16, 26) dudit rabat latéral comporte au moins une partie incurvée (15, 16) s'étendant entre ledit bord intérieur (23, 24) et ledit bord extérieur (19, 20), dans lequel :
chacune desdites sections élastiques (33, 34) comprend un stratifié élastique comportant un matériau élastique (35) adjacent à et attaché fixement à deux couches (36, 37) d'un matériau fibreux non élastique, et dans lequel
lesdites deux couches (36, 37) d'un matériau fibreux non élastique s'étendent sur toute la superficie dudit rabat latéral (10, 11), tandis que le matériau élastique (35) ne s'étend que dans la zone qui doit former la section élastique (33, 34).

2. Article absorbant selon la revendication 1, **caractérisé en ce que** ladite section élastique (33, 34) s'étend, dans le sens longitudinal, substantiellement sur toute la distance qui sépare le bord supérieur (12, 13) dudit rabat latéral (10, 11) du point d'extrémité inférieure (17, 18) dudit bord extérieur (19, 20).

3. Article absorbant selon la revendication 1, **caractérisé en ce que** ladite partie incurvée (15, 16) s'étend sur une partie de la distance qui sépare ledit bord intérieur (23, 24) du point d'extrémité inférieure (17, 18) dudit bord extérieur (19, 20).

4. Article absorbant selon l'une quelconque des revendications précédentes, **caractérisé en ce que** chacun desdits rabats latéraux (10, 11) est attaché de manière fixe au niveau d'une partie de bord latéral longitudinal (21, 22) de ladite feuille supérieure (6) et/ou de ladite feuille arrière (5).

5. Article absorbant selon la revendication 4, **caractérisé en ce que** ledit bord supérieur (12, 13) est substantiellement aligné avec un bord supérieur (14) de ladite feuille supérieure (6) et/ou de ladite feuille arrière (5).

6. Article absorbant selon l'une quelconque des revendications précédentes, **caractérisé en ce que** ledit stratifié élastique comportant une lame de matériau élastique (35) est attaché de manière fixe dans un état allongé au moins latéralement entre deux lames (36, 37) d'un matériau fibreux non élastique.

7. Article absorbant selon l'une quelconque des revendications précédentes, **caractérisé en ce qu'**au moins un élément élastique (27, 28), formant partie d'une partie de jambe élastique sur un côté respectif dudit article absorbant, s'étend dans la section non élastique (29, 30) dudit rabat latéral sur ledit côté respectif.

8. Article absorbant selon l'une quelconque des revendications précédentes, **caractérisé en ce que** ladite section élastique (10, 11) est substantiellement rectangulaire, et **en ce que** ladite section élastique (10, 11) comporte une limite supérieure (38), et **en ce que** ladite limite supérieure (38) est alignée avec un bord supérieur (39) dudit rabat latéral (10, 11).
